(19) Europäisches Patentamt
European Patent Office
Office européen des brevets

(11) Veröffentlichungsnummer: **0 091 645**
**B1**

(12) **EUROPÄISCHE PATENTSCHRIFT**

(45) Veröffentlichungstag der Patentschrift: **25.02.87**

(51) Int. Cl.⁴: **C 07 H 13/04**, C 07 H 13/00, A 61 K 31/70

(21) Anmeldenummer: **83103325.3**

(22) Anmeldetag: **06.04.83**

(54) **N-glycosylierte Carbonsäureamid-Derivate, Verfahren zu ihrer Herstellung sowie ihre Verwendung zur Beeinflussung der körpereigenen Abwehr.**

(30) Priorität: **14.04.82 DE 3213650**

(43) Veröffentlichungstag der Anmeldung: **19.10.83 Patentblatt 83/42**

(45) Bekanntmachung des Hinweises auf die Patenterteilung: **25.02.87 Patentblatt 87/9**

(84) Benannte Vertragsstaaten: **AT BE CH DE FR GB IT LI LU NL SE**

(56) Entgegenhaltungen:
**DE-B-1 261 861**
**US-A-4 228 274**

**CHEMICAL ABSTRACTS, Band 87, Nr. 25, 19. Dezember 1977, Seite 768, Nr. 201958p, Columbus, Ohio, USA. E. MASCIORINI et al.: "Synthesis of some N-methyl-N-acyl-aldopyranosylamines and their per-0-acyl derivatives"**
**CHEMICAL ABSTRACTS, Band 78, Nr. 21, 28. Mai 1973, Seiten 415-416, Nr. 136597t, Columbus, Ohio, USA. M. M. VIGDORCHIK et al.: "N-Glycosides of indolylalkylamines. III. PMR spectra of N-glucosides of N-acetyltryptamines and N-acetyl-naphthylalkylamines"**
**CHEMICAL ABSTRACTS, Band 76, Nr. 15, 20. April 1972, Seite 441, Nr. 86057c, Columbus, Ohio, USA. D. BUNDLE et al.: "Synthesis of 2-acetamido-6-0-(2-acetamido-2-deoxy-bete-D-glucopyranosyl)-2-deoxy-D-glucopyranose"**
**CHEMICAL ABSTRACTS, Band 90, Nr. 9, 26. Februar 1979, Seite 539, Nr. 72406t, Columbus, Ohio, USA, M. GOMEZ GUILLEN et al.: "Synthesis of 2-(alkylamino)-2-deoxyheptononitriles. II. Synthesis of 2-deoxy-2-(propyl (or isopropyl)amino)heptononitriles"**
**CHEMICAL ABSTRACTS, Band 90, Nr. 3, 15. Januar 1979, Seite 695, Nr. 23464b, Columbus, Ohio, USA, M. GOMEZ GUILLEN et al.: "Synthesis of 2-(alkylamino)-2-deoxyheptononitriles. III. Synthesis of 2-deoxy-**

(73) Patentinhaber: **BAYER AG, Konzernverwaltung RP Patentabteilung, D-5090 Leverkusen 1 Bayerwerk (DE)**

(72) Erfinder: **Stadler, Peter, Dr., c/o Ames Div. Miles Lab. Inc. P.O. Box 70, Elkhart Indiana 46515 (US)**
Erfinder: **Lockhoff, Oswald, Dr., Bamberger Strasse 3, D-5090 Leverkusen 1 (DE)**
Erfinder: **Opitz, Hans- Georg, Dr., Steinberger Weg 52, D-5600 Wuppertal 17 (DE)**
Erfinder: **Schaller, Klaus, Dr., Am Sonnenschein 38, D-5600 Wuppertal 1 (DE)**

(56) Entgegenhaltungen: (Fortsetzung)
**2-(ethylamino)heptononitriles"**
**CHEMICAL ABSTRACTS, Band 87, Nr. 11, 12. September 1977, Seite 621, Nr. 85185h, Columbus, Ohio, USA. G. CHETTUR et al.: "A new chemical synthesis of 2-amino-(N-D-ribofuranosyl)acetamide 5'-phosphate"**
**CHEMICAL ABSTRACTS, Band 89, Nr. 21, 20. November 1978, Seite 636, Nr. 180264h, Columbus, Ohio, USA. R. T. LEE et al.: "Some synthetic inhibitors of beta-D-glucosiduronase"**
**CHEMICAL ABSTRACTS, Band 76, Nr. 25, 19. Juni 1972, Nr. 150634t, Columbus, Ohio, USA. S. BLUMENBERG et al.: "Use of 1-amino-L-fucose bound to Sepharose in the isolation of L-fucose-binding proteins"**
**CHEMICAL ABSTRACTS, Band 95, Nr. 3, 20. Juli 1981, Seite 740, Nr. 25452y, Columbus, Ohio, USA. B. A. KLYASHCHITSKII et al.: "Covalent addition of biologically active agents to polymers. IV. Synthesis of ("affinity") adsorbents containing L-fucose derivatives"**
**CHEMICAL ABSTRACTS, Band 83, Nr. 1, 7. Juli 1975, Seite 307, Nr. 3348u, Columbus, Ohio, USA. N. HARPAZ et al.: "Alpha-Galactosidase"**
**CHEMICAL ABSTRACTS, Band 89, Nr. 15, 9. Oktober 1978, Seite 624, Nr. 129847y, Columbus, Ohio, USA. S. HIRANO et al.: "Preparation of some novel N-acyl derivatives of 2-acetamido-3,4,6-trei-0-acetyl-2-deoxy-beta-D-glucopyranosylamine"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft Verbindungen der allgemeinen Formel (I)

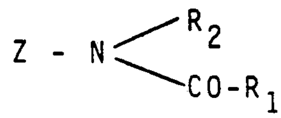

worin

Z einen über das anomere Kohlenstoffatom gebundenen Maltoserest oder Monosaccharidrest,

$R_1$ einen gegebenenfalls substituierten geradkettigen oder verzweigten, gesättigten Alkylrest mit 9 bis 21 C-Atomen oder einen ein- oder zweifach ungesättigten Alkenylrest mit 7 bis 21 C-Atomen und

$R_2$ einen geradkettigen oder verzweigten, gesättigten oder ein- oder mehrfach ungesättigten, gegebenenfalls durch bis zu 5 Sauerstoffatome enthaltende Gruppen oder Halogenatome substituierten Alkyloder Aralkylrest mit bis zu 30 C-Atomen oder einen Alkoxyalkylrest oder (Alkoxy-alkoxy)-alkylrest mit bis zu 30 O- und C-Atomen bedeuten.

Beispielhaft für gesättigte Reste seien hier genannt n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Docosyl, Ethylpentyl, Methyldecyl, i-Propyldecyl und Methyltridecosyl.

Ungesättigte Reste sind beispielsweise 1-Decenyl, 5-Decenyl, 9-Decenyl, 8-Heptadecenyl, 1,3-Butadienyl, 1,3-Pentadienyl, 1,4-Pentadienyl, 2,4-Pentadienyl, 8,11-Heptadecandienyl und 8,11,14-Heptadecantrienyl. Im allgemeinen sind die längerkettigen, ungesättigten Reste bevorzugt, speziell die ein- oder zweifach ungesättigten Alkenyle mit 7 - 21 C-Atomen.

Die ungesättigten Kohlenwasserstoffreste können dabei als reine cis- oder trans-Isomere oder auch als Isomerengemische vorliegen.

$R_2$ in Formel (I) steht für einen geradkettigen oder verzweigten, gesättigten oder einfach bzw. mehrfach ungesättigten Alkyl- oder Aralkylrest mit bis zu 30 Kohlenstoffatomen. Einzelne Wasserstoffatome in den Alkyl- oder Aralkylresten können auch durch bis zu 5 Sauerstoff enthaltende Gruppen oder Halogenatome substituiert sein.

Beispiele, in welchen $R_2$ in Formel (I) für einen geradkettigen oder verzweigten, gegebenenfalls einfach oder mehrfach ungesättigten Alkylrest steht, sind u.a. die für $R_1$ genannten.

Besonders seien genannt Methyl, Propyl, Hexyl, Decyl, Undecyl, Dodecyl, Tridecyl, Tetradecyl, Pentadecyl, Hexadecyl, Heptadecyl, Octadecyl, Nonadecyl, Docosyl, Muricyl, Ethylhexyl, Isobutyl, Propenyl, Octenyl, Hexadienyl, Docosenyl, Dimethylhexenyl und 2-(Cyclohexyl)-ethyl. Die ungesättigten Kohlenwasserstoffe können als reine cis- oder trans-Isomere oder als Isomerengemisch vorliegen. Durch Sauerstoffatome enthaltende Gruppen substituierte Kohlenwasserstoffreste $R_2$ sind beispielsweise Hydroxypropyl und Hydroxydimethyloctyl. Alkoxyalkylreste oder (Alkoxy-alkoxy)alkylreste sind beispielsweise Methoxybutyl oder Butoxypropyl oder Ethoxyethoxyethyl. Als Beispiel für einen halogensubstituierten Kohlenwasserstoffrest sei Trifluormethylethyl genannt.

Aralkyl für $R_2$ in Formel I ist zum Beispiel Aryl-niedrigalkyl wie Benzyl, Phenethyl oder Phenylhexyl, wobei der Phenylkern gegebenenfalls einfach oder mehrfach, vorzugsweise einoder zweifach, zum Beispiel durch Niederalkyl, Trifluormethyl, Halogen, Hydroxy oder Niedrigalkoxy substituiert sein kann.

Unter Niedrigalkyl oder -alkoxy werden solche Reste verstanden, die 1 bis 5, vorzugsweise 1 bis 3 C-Atome enthalten.

Z in Formel I bedeutet einen Maltoserest oder Monosaccharidrest, der bei den erfindungsgemäßen Verbindungen immer über das anomere Kohlenstoffatom an den Amidstickstoff gebunden ist, wobei darunter erfindungsgemäß Monosaccharidreste verstanden werden, in denen gegebenenfalls einer oder mehrere Hydroxylgruppen durch Aminogruppen, Acylamidogruppen, Azidogruppen, Wasserstoff, Nitro, Thiolgruppen oder Niedrigalkoxy oder Halogen ersetzt sein können und die auch in Form der entsprechenden Ulosen, Ulosederivate, Uronsäuren oder von Uronsäurederivaten vorliegen können.

Die Monosaccharidreste Z in Formel (I) liegen erfindungsgemäß bevorzugt in der Pyranosyl- oder der Furanosylform vor, und sind bevorzugt aus Tetrosen, Pentosen, Hexosen und Heptosen aufgebaut.

Erfindungsgemäße Beispiele für Monosaccharidreste sind Glucopyranosyl-, Galactopyranosyl-, Mannopyranosyl-, Glucofuranosyl-, Ribofuranosyl, Arabinopyranosyl- oder auch Lyxopyranosyl- oder auch D-Glycero-D-gluco-heptopyranosylreste.

Als Beispiele für Monosaccharidreste, in denen eine oder mehrere OH-Gruppen durch Aminogruppen,

Acylamidogruppen, Azidogruppen, Wasserstoff, Nitro, Thiolgruppen, Niedrigalkoxy oder Halogen ersetzt sind, seien genannt 2-Acetylamido-2-desoxyglucopyranosyl-, 2-Amino-2-desoxy-glucopyranosyl-, 4-Azido-4-desoxy-glucopyranosyl-, 4-Stearoylamido-4-desoxy-D-glucopyranosyl-, 4-Dodecoylamido-4-desoxy-D-glucopyranosyl-, 6-Hexa-decanoylamido-6-desoxy-D-galactopyranosyl-, 2,6-Diamino-2,6-didesoxyglucopyranosyl-, 6-Desoxymannopyranosyl-, 2-Desoxyribofuranosyl-, Fucosyl, 5-Fluor-5-desoxy-ribofuranosyl-, 6-0-Methylglucopyranosyl, 6-Desoxy-6-thio-glucopyranosyl, 3-Desoxy-3-nitrogalactopyranosyl.

Liegen die Glykosylreste in Form von Uronsäuren oder Uronsäurederivaten vor, so handelt es sich um Glykuronsäuren mit freier Carboxylgruppe oder mit durch Alkyl veresteter Carboxylgruppe oder um Glykuronamidderivate mit unsubstituiertem oder substituiertem Stickstoffatom. Beispiele für entsprechende Zucker sind Galacturonsäure, Glucuronsäuremethylester, Glucuronsäureamid oder auch N-Dodecylglucuronamid.

Die Verbindungen der Formel I enthalten mehrere chirale C-Atome und liegen als optische reine Diasteromere oder als Diasteromerengemische vor. Die erfindungsgemäßen Verbindungen der Formel I sind also Carbonsäureamide oder N-alkylierte bzw. N-Aralkylierte Carbonsäureamide, die jeweils am Amidstickstoff N-glykosidisch-d.h. also über das anomere Kohlenstoffatom gebunden- einen einfachen oder modifizierten Monosaccharid- oder Maltoserest tragen.

Ganz besonders bevorzugte Verbindungen sind die durch Ausführungsbeispiele belegten, insbesondere die der Beispiele 9, 10, 11, 12, 13, 15, 16, 20, 22, 30, 31, 32, 34, 36, 37, 39, 40, 42, 43, 45, 46, 47, 50, 51, 52, 53 und 54.

Die Erfindung betrifft auch Verfahren zur Herstellung der Verbindungen der Formel I. Hierbei setzt man die von Z in Formel I umfaßten Zucker entweder in freier, d.h. ungeschützter Form, oder in Form geschützter, gegebenenfalls aktivierter Derivate zunächst mit einer Aminoverbindung $R_2$-$NH_2$, entweder in freier Form oder in Form eines geeigneten Säureadditionssalzes mit der vorstehend beschriebenen Bedeutung für $R_2$, um und acyliert anschließend das dabei erhaltene Glykosylamin mit einemwie bei Acylierungsreaktionen üblich - aktivierten, an funktionellen Gruppen gegebenenfalls geschützten, Carbonsäurederivat, spaltet in dem so erhaltenen Reaktionsprodukt gegebenenfalls vorhandene Schutzgruppen ab und erhält auf diese Weise die erfindungsgemäßen Verbindungen der Formel I, die falls erforderlich, durch Chromatograpbie, Umkristallisieren, Extraktion o.ä. gereinigt werden können.

Als Aminoschutzgruppen kommen solche in der Zucker- und Peptidchemie üblicherweise verwendeten Gruppen in Frage (siehe zum Beispiel HOUBEN-WEYL, Methoden der organischen Chemie, Band XV, Georg Thieme Verlag, Stuttgart, 1974), die einerseits unter den gegebenen Reaktionsbedingungen stabil sind, die andererseits aber nach der Herstellung des betreffenden N-Glykosids und dessen anschließender Acylierung mit einem Carbonsäurederivat $R_1$-CO-X, bei der vorstehend angegebenen Bedeutung für $R_1$, so selektiv wieder abgespalten werden können, daß das gewünschte Endprodukt der Formel I erhalten wird, d.h. ohne daß die im Endprodukt der Formel I enthaltende Acylaminogruppe nach gespalten wird. Bevorzugte Beispiele sind Acylgruppe vom Typ

$$-\underset{\underset{O}{\parallel}}{C}-B$$

Wobei B Trichlormethyl oder Trifluormethyl bedeutet oder vom Typ

$$-\underset{\underset{O}{\parallel}}{C}-O-E$$

wobei E z.B. für Trichlorethyl oder tertiär-Butyl steht oder auch Sulfenylgruppen vom Typ
- S - G
wobei G für Phenyl, substituiertes Phenyl oder Di- oder Triphenylmethyl steht und 'substituiertes Phenyl' einen Phenylrest bezeichnet, der durch einen bis drei Substituenten aus der Reihe Nitro, Niedrigalkyl oder der durch 1 bis 5 Halogenatome, vorzugsweise Chloratome, substituiert ist. Als Beispiele seien der 2,4,5-Trichlorphenylsulfenyl und der o-Nitrophenylsulfenylrest genannt.

Die Einführung dieser Schutzgruppen in Aminoverbindungen und ihre spätere Abspaltung zur Freisetzung der gewünschten Aminogruppen sind bekannt und beispielsweise in der oben zitierten Literaturstelle beschrieben.

In einer anderen Ausführungsform des Verfahrens zur Herstellung von solchen Produkten der Formel I in denen im Glykosylrest Z eine oder mehrere freie Aminogruppen vorhanden sind, setzt man Zuckerderivate Z-OH als Ausgangsprodukte ein, in welchen die Aminogruppe oder die Aminogruppen zunächst in Form von Azidoresten, d.h. also in verkappter Form vorliegen. Im abschließenden Schritt der Herstellung der

Verbindungen der Formel I werden diese Azidogruppen reduktiv in an sich bekannter Weise in Aminogruppen umgewandelt, wobei darauf geachtet wird, daß solche Reduktionsmittel verwendet werden, die andere gegebenenfalls im Molekül vorhandene reduktionsempfindliche Gruppen nicht angreifen.

Entsprechende Azidozucker und deren Herstellung sind bekannt (siehe zum Beispiel Methods in Carbohydrate Chemistry, Vol. I, 242 - 246 Academic Press, 1962 New York a. London). Zur Reduktion können verwendet werden, Hydriddonatoren wie z.B. Natriumboranat oder Lithiumalanat, katalytisch erregter Wasserstoff oder auch Triphenylphosphin in Methanol/Amoniak/Pyridin oder auch Schwefelwasserstoff oder Mercaptane in protischen Solventien.

Als Lösungsmittel können alle gängigen organischen Solventien, vorzugsweise niedrige Alkanole, oder aber auch Wasser oder wäßrige Alkanole verwendet werden.

Die Reaktionen werden gegebenenfalls unter Zusatz von organischen Säuren wie zum Beispiel Essigsäure oder anorganischen Säuren wie zum Beispiel Schwefelsäure oder unter Zusatz organischer Basen wie zum Beispiel Pyridin oder anorganischer Basen wie zum Beispiel Ammoniak durchgeführt. Man arbeitet bei Temperaturen zwischen 0 und 120°C vorzugsweise 10°C bis 40°C, gegebenenfalls unter erhöhtem Druck und/oder Intergas.

Für den Fall, daß in den erfindungsgemäßen Endprodukten der Formel I im Kohlenhydratteil Z eine oder mehrere OH-Gruppen durch eine oder mehrere Acylamidogruppen ersetzt sind, werden die Zucker Z-OH von vornherein in Form der entsprechenden Acylamidozucker eingesetzt. Die Acylamidozucker werden dann am anomeren Zentrum mit den obigen angegebenen Aminen zunächst zu den entsprechenden Acylamidoglycosylaminen umgesetzt und im zweiten Reaktionsschritt an der C-1 Aminogruppe des Zuckerteils acyliert zu N-(Acylamidoglycosyl)amiden der Formel I.

Eine andere Verfahrensweise zur Darstellung von Verbindungen der Formel I, in der Z für einen mit einer oder mehreren Acylamidogruppen substituierten Zuckerrest steht, besteht darin, daß man Aminodesoxyzucker, die die Aminogruppe an einem anderen als dem anomeren Kohlenstoffatom tragen, mit Aminen der Formel $R_2$-$NH_2$ umsetzt zu (Amino-desoxy-glycosyl)aminen, welche dann im zweiten Reaktionsschritt zweifach oder gegebenenfalls mehrfach acyliert werden zu N(Acylamidoglycosyl)-amiden der Formel I

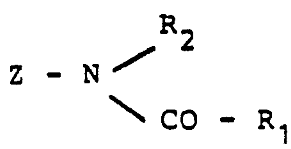

Ferner ist es auch möglich, die Verbindungen der Formel I, in der Z für einen mit einer oder mehreren Acylamidogruppen substituierten Zuckerrest steht, zu erhalten, indem man in Derivaten der Formel I, in der Z zunächst einen durch eine oder mehrere temporäre Amino-Schutzgruppen vom vorstehend beschriebenen Typ

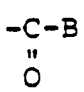 , 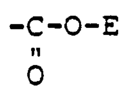 oder -S-G

blockierten Aminozucker darstellt, die temporäre Amino-Schutzgruppen nach den üblichen Methoden abspaltet zu den entsprechenden N-(Aminodesoxyglycosyl)-amiden und diese dann umsetzt mit aktivierten Carbonsäurederivaten zu den entsprechenden N-(Acylamidoglycosyl)-amiden der Formel I

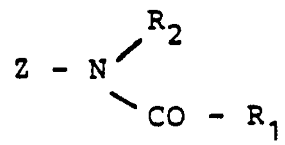

Eine andere Verfahrensweise zur Darstellung von N-(Acylamidoglycosyl)-amiden der Formel I besteht darin, daß man N-(Azidoglycosyl)-amide der Formel I nach den üblichen Methoden umsetzt zu den N-(Aminoglycosyl)-amiden der Formel I und diese dann mit aktivierten Carbonsäurederivaten acyliert zu N-(Acylamidoglycosyl)-amiden der Formel I.

Der erste Verfahrensschritt bei der Herstellung der erfindungsgemäßen Verbindungen der Formel I ist somit die Umsetzung eines Zuckers Z-OH, mit einem amin des Typs $R_2$-$NH_2$ am anomeren Kohlenstoffatom unter Wasserabspaltung zu dem betreffenden Glykosylamin.

Amine $R_2$-$NH_2$, die bei Raumtemperatur flüssig sind, können mit dem Zucker direkt, d.h. ohne Lösungsmittel umgesetzt werden. Hierbei arbeitet man bei Temperaturen zwischen 0°C und 100°C vorzugsweise bei 25°C bis 70°C. Als Katalysatoren sind Mineralsäuren wie zum Beispiel Salzsäure, Schwefelsäure oder Salpetersäure oder kurzkettige Carbonsäuren wie Essigsäure oder Propionsäure geeignet, die man in Mengen von 0,001 bis 0,05 Äquivalenten einsetzt.

In jeden Fall ist es möglich, und bei Aminen $R_2$-$NH_2$, die bei Raumtemperatur fest sind auch zu bevorzugen, die Herstellung der Glykosylamine in Gegenwart eines Lösungsmittels durchzuführen. Man arbeitet dann vorzugsweise in Gegenwart eines unter den Reaktionsbedingungen inerten Verdünnungsmittels, welches vorzugsweise so beschaffen ist, daß sich zumindest entweder die Reaktionspartner oder das Reaktionsprodukt in ihm lösen.

In Frage kommen Alkohole wie Methanol, Ethanol, Propanol-1 und Propanol-2, Ether wie Tetrahydrofuran und Dioxan, sowie auch Dimethylformamid, wobei - außer bei der Verwendung der Alkohole - der Zusatz von Wasser zu bevorzugen ist. Darüber hinaus ist vorzugsweise bei kurzkettigen Aminen $R_2$-$NH_2$ auch Wasser allein als Lösungsmittel geeignet. Es kann auch von Vorteil sein, die Alkanole im Gemisch mit Wasser zu verwenden.

Die Reaktionstemperaturen bei Verwendung von Lösungsmitteln bei der Herstellung der Glykosylamine liegen zwischen -10°C und 120°C vorzugsweise zwischen 30°C und 70°C.

Das betreffende Verdünnungsmittel kann wahlweise vor oder während der Reaktion zugegeben werden. Bei langkettigen Aminen $R_2$-$NH_2$ ist eine Zugabe vor der Reaktion zu bevorzugen.

Die wie vorstehend beschrieben herstellten Glykosylamine kristallisieren entweder direkt oder nach Abkühlen aus und können durch Zusatz geeigneter, vorzugsweise weniger polarer Hilfslösungsmittel wie Aceton, Diethylether, Cyclohexan, Essigester oder Petrolether, gegebenenfalls unter Kühlung, ausgefällt oder zur Kristallisation gebracht werden, gegebenenfalls vorhandenes überschüssiges Amin $R_2$-$NH_2$ kann durch Waschen oder Umkristallisieren des Produktes auf an sich bekannter Weise entfernt werden.

Der zweite Verfahrensschritt bei der Herstellung der erfindungsgemäßen Verbindungen der Formel I ist die selektive N-Acylierung eines wie vorstehend beschrieben erhaltenen Glykolsylamins mit einem Carbonsäurederivat der Formel $R_1$ - CO - X mit der vorstehend angegebenen Bedeutung von $R_1$ und X. Als an sich bekannter Carbonsäurederivate R-CO-X sind zu bevorzugen Anhydride, aktivierte Ester und Säurehalogenide, vorzugsweise Chloride.

Diese Acylierungsmittel werden vorzugsweise in Gegenwart eines Verdünnungsmittels mit den Glykosylaminen umgesetzt, in welchem die Reaktionspartner vollständig oder auch nur teilweise gelöst sind.

Es kommen organische oder anorganische Solventien in Frage vorzugsweise solche, die unter den Reaktionsbedingungen Nebenreaktionen möglichst herabsetzen oder verhindern. Man kann sowohl in organischen Lösungsmitteln wie Ethern, z.B. Tetrahydrofuran und Dioxan, oder Alkoholen z.B. Ethanol und Propanol, oder Ketonen, z.B. Aceton oder Methylethylketon, oder in Dimethylformamid, Essigester oder Pyridin als auch in Mischungen dieser Lösungsmittel untereinander und/oder mit Wasser arbeiten. Die Verwendung von wasserfreien Solventien ist im allgemeinen zu bevorzugen.

Die Acylierungsmittel $R_1$-CO-X werden in 1 - 10 Äquivalenten, bezogen auf Glykosylamin eingesetzt, wobei die Verwendung von 1 - 3 Äquivalenten zu bevorzugen ist.

Die Acylierungsreaktionen können, vorzugsweise bei Verwendung von Säurehalogeniden und -anhydriden, in Gegenwart basischer Hilfsstoffe durchgeführt werden. Verwendet werden können alle in der organischen Synthese üblichen basischen Verbindungen wie z.B. tertiäre aliphatische oder auch aromatische Amine oder aber Alkali- und Erdalkalihydroxide bzw. -carbonate wie Natronlauge, Natriumcarbonat oder Calciumcarbonat.

Die Acylierungen werden bei Temperaturen zwischen etwa -30°C und +80°C, vorzugsweise zwischen -10°C und +20°C, durchgeführt.

Die auf dese Weise erhaltenen Amide werden nach an sich bekannten Verfahren in Form von kristallinen oder amorphen Feststoffen oder als zähe Sirupe isoliert und, wenn notwendig, durch Umkristallisation, Chromatographie, Extraktion usw. gereinigt.

Im Falle von Verbindungen mit geschützten Aminogruppen im Glykosylteil werden die Schutzgruppen in an sich bekannter Weise abgespalten.

Das folgende Formelschema soll eine der bevorzugten Ausführungsformen der erfindungsgemäßen Darstellung von Verbindungen der Formel I beispielhaft erläutern:

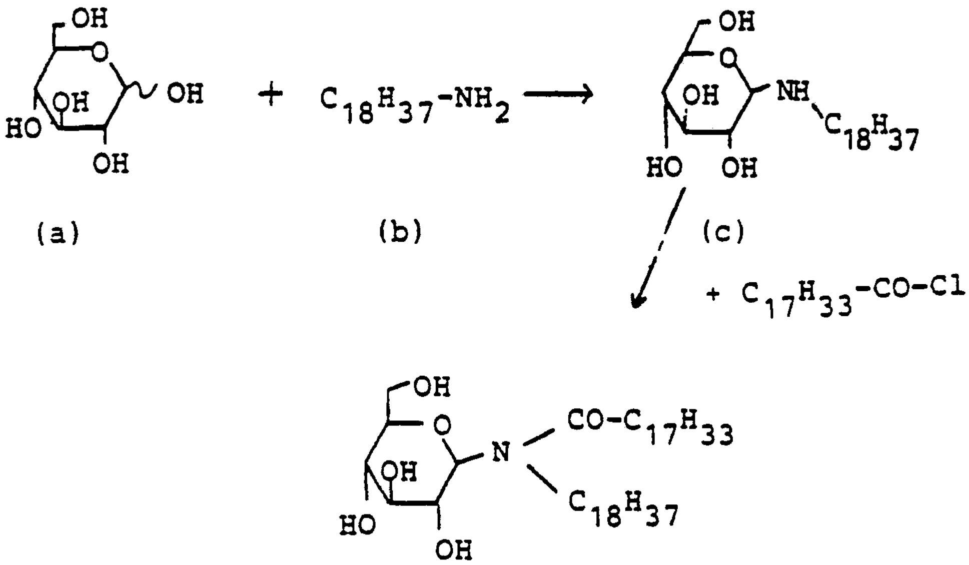


I

Im ersten Verfahrensschritt wird Glucose (a) mit Octadecylamin (b) zu N-Octadecyl-β-D-Glucopyranosylamin (c) umgesetzt, das im zweiten Verfahrensschritt mit Ölsäurechlorid zu N-Octadecyl-N-oleyl-β-D-glucopyranosylamin (I) acyliert wird.

Die erfindungsgemäßen Verbindungen der Formel (I) werden auf die vorstehend beschriebene Weise in hohen Ausbeuten erhalten

Die Verbindungen der Formel (I) können auch hergestellt werden durch Acylierung solcher Glykosylamine, in denen Z einen chemisch veränderten Zuckerrest bezeichnet, in welchem alle im Molekül vorhandenen Hydroxylgruppen mit einer leicht abspaltbaren Schutzgruppe geschützt sind.

Leicht abspaltbare Schutzgruppen sind aus der Peptidchemie bzw. Zuckerchemie bekannt.

Zum Gegenstand der Erfindung gehören auch Salze der Verbindungen der Formel I. Dabei handelt es sich in erster Linie um üblicherweise pharmazeutisch verwendbare, nicht -toxische Salze, z.B. Alkalimetall- oder Ammoniumsalze.

Die Verbindungen der vorliegenden Erfindung weisen wertvolle pharmakologische Eigenschaften - insbesondere eine ausgeprägte Abwehr-steigernde Wirkung - auf. Es wurde gefunden, daß die Verbindungen der vorliegenden Erfindung die Antikörpersynthese des Immunssystems steigern und darüber hinaus die unspezifische Wirtseigene Abwehr verstärken. Dies kann anhand der nachstehend beschriebenen Versuchsanordnung gezeigt werden.

Potenzierung der humoralen Immunität gegen Schafserythrocyten (SE).

Es ist experimentell möglich, den gesamten Verlauf der Antikörpersynthese in vitro ablaufen zu lassen. Hierzu werden Milzzellkulturen der Maus mit Schafserythrocyten (SE) immunisiert. 5 Tage später werden die Anti-SE-Antikörperbildenden Zellen bestimmt. Verbindungen der vorliegenden Erfindung - wie in Tabelle 1 gezeigt - sind überraschenderweise in der Lage, dosisabhängig im Bereich 1 - 30 µg/ml die Zahl der antikörperbildenden Zellen zu steigern.

**Tabelle 1**

Wirkung von ausgewählten N-glycosylierten Carbonsäureamid-Derivaten auf die Antikörpersynthese gegen Schaferythrocyten in vitro.

| Verbindung gemäß Beispiel | Antikörper sezernierende Zellen/Kultur in Abhängigkeit von der Dosis (µg/ml) | | | | |
|---|---|---|---|---|---|
| | 0 | 1 | 3 | 10 | 30 |
| 12 | 2620 | 2840 | 4040 | 6760 | 12330 |
| 15 | 2030 | 2350 | 3660 | 7580 | 10300 |
| 16 | 1390 | 2090 | 2550 | 8760 | 12680 |
| 32 | 2700 | 3680 | 4850 | 5200 | 8160 |
| 20 | 2760 | 4000 | 5740 | 6920 | 10600 |

Potenzierung der humoralen Immunität in vivo: Steigerung der Antikörperproduktion gegen Schafserythrocyten bei der Maus.

NMRI-Mäuse wurden durch intraperitoneale (i.p.) Injektion von $10^7$ SE immunisiert. 5 Tage später wurden die Milzen entnommen und die Zahl der Anti-SE-Antikörper sezernierender Lymphocyten bestimmt. In einer zweiten Versuchsanordnung wurde der Titer der hämagglutinierenden Antikörper im Serum der Tiere bestimmt. In der verwendeten Dosis sind die SE für die Empfängertiere suboptimal, d.h. sie vermögen nur eine geringe Zahl von Lymphocyten zur Antikörpersynthese anzuregen. Die zusätzliche Behandlung der Tiere mit Verbindungen der vorliegenden Erfindung ist in der Lage, bei einmaliger intraperitonealer oder subcutaner (s.c.) Applikation von 1,0 - 100 mg/kg die Zahl der antikörperbildenden Zellen um den Faktor 5 bis 10 über den Kontrollwert zu steigern und den Antikörpertiter im Serum der Tiere signifikant zu erhöhen.

Der immunstimulierende Effekt der genannten Verbindungen ist im Gegensatz zu anderen bakteriellen Immunstimulantien (z.B. LPS aus grannegativen Mikroorganismen) antigenabhängig, d.h. die Injektion der neuen Verbindungen hat nur in SE-immunisierten, nicht aber in nichtimmunisierten mäusen eine Erhöhung der Anti-SE-Titer zur Folge.

Potenzierung der humoralen Immunität in vivo: Steigerung der Antikörperproduktion gegen Ovalbumin.

NMRI-Mäuse werden durch intraperitoneale Injektion von 50 µg Ovalbumin am Tag 0 immunisiert. 7, 14 und 21 Tage später werden Serumproben entnommen und auf ihren Gehalt an anti-Ovalbumin Antikörper mittels passiver Hämagglutination untersucht. In der verwendeten Dosis ist Ovalbumin für die Mäuse subimmunogen, d.h. es vermag keine oder nur eine ganz geringfügige Produktion von Antikörpern auszulösen. Eine Behandlung der Mäuse mit bestimmten immunpotenzierenden Stoffen vor oder nach der Antigengabe führt zu einem Anstieg der Antikörpertiter im Serum. Der Effekt der Behandlung wird durch den erreichten Scorewert, d.h. durch die Summe der $\log_2$ Titerdifferenzen an den drei Blutungstagen ausgedrückt.

In diesem Test sind die Verbindungen der Formel I in der Lage, bei intraperitonealer oder subcutaner Applikation von 1-100 mg/kg am Tage der Immunisierung mit Ovalbumin der Antikörperproduktion gegen Ovalbumin signifikant zu steigern.

Aktivierung von Makrophagen.

Makrophagen spielen bei unspezifischen Abwehrprozessen eine zentrale Rolle. Auf ein Antigen hin reagieren sie charakteristischerweise mit einem gesteigerten Stoffwechsel (Makrophagen-Aktivierung), der sich vor allem durch erhöhte sekretorische Leistungen nachweisen läßt. Es konnte gezeigt werden, daß die genannten Verbindungen bevorzugt mit einem Antigen, wie z.B. Candia albicans, eine Steigerung der Makrophagen-Aktivierung bewirken. Gemessen wurde die Freisetzung von cytotoxisch wirkenden Superoxyd. Es zeigte sich, daß die Verbindungen der genannten Art die Produktion von Superoxid wenigstens um den Faktor 2 zu steigern vermögen.

Verträglichkeit

Obwohl Verbindungen der beschriebenen Art ihre potenzierende Wirkung an der Maus beispielsweise bereits nach einer Einzeldosis von 10 mg/kg i.p., oder peroral entfalten, werden auch bei Applikation von 100 mg/kg keine toxischen Effekte beobachtet. Die genannten Stoffe verfügen deshalb über eine gut Verträglichkeit.

Die erfindungsgemäßen Verbindungen haben die Fähigkeit, einerseits bei Mischung mit einem Antigen

dessen Immunogenität zu erhöhen, andererseits bei systemischer Applikation die immunologische Reaktivität des behandelten Organismus zu steigern. Dabei sind die genannten Stoffe in der Lage, die für die Antikörperbildung verantwortlichen Lymphocyten zu aktivieren.

Die neuen Verbindungen können somit als Adjuvantien in Mischung mit Impfstoffen dazu benutzt werden, den Impferfolg zu verbessern und den durch Immunität vermittelten Infektionsschutz gegenüber bakteriellen, viralen oder parasitären Erregern zu steigern.

Weiterhin eignen sich die beschriebenen Verbindungen in Mischung mit verschiedensten Antigenen als Adjuvantien bei der experimentellen und industriellen Herstellung von Antiseren für Therapie und Diagnostik.

Darüber hinaus können die neuen Verbindungen auch ohne gleichzeitige Antigenzufuhr dazu benützt werden, bereits unterschwellig ablaufende Abwehrreaktionen bei Mensch und Tier zu fördern. Die Verbindungen eignen sich demnach besonders für die Stimulation der körpereigenen Abwehr, z.B. bei chronischen und akuten Infektionen oder bei selektiven (antigenspezifischen) immunologischen Defekten, sowie bei angeborenen, aber auch bei erworbenen allgemeinen (d.h. nicht antigenspezifischen) immunologischen Defektzuständen, wie sie im Alter, im Verlauf schwerer Primärerkrankungen und vor allem nach Therapie mit ionisierenden Strahlen oder mit immunosuppressiv wirkenden Stoffen auftreten. Die genannten Stoffe können somit vorzugsweise auch in Kombination mit antiinfektiösen Antibiotika, Chemotherapeutika oder anderen Heilverfahren verabreicht werden, um immunologischen Schädigungen entgegenzuwirken. Schließlich sind die beschriebenen Stoffe auch zur allgemeinen Prophylaxe von Infektionskrankheiten bei Mensch und Tier geeignet.

Die erfindungsgemäßen Verbindungen erhöhen die überlebensrate im Tiermodell der systemischen Mäusecandidose.

**Versuchsbeschreibung**

Mäuse vom Typ SFF-$CF_1$ wurden intravenös mit 2 - 6 x $10^5$ logarithmisch wachsenden Zellen von Candida albicans, suspendiert in physiologischer Kochsalzlösung, infiziert. Beginnend mit dem 3. Tag post infectionem werden bei unbehandelten Kontrolltieren die ersten Krankheitssymptome erkennbar. Bis zum 5. Tag sterben die ersten Tiere an akutem Nierenversagen und bis zum 14. Tag post infectionem sind in der Regel mehr als 80 % der unbehandelten Tiere gestorben. In diesem Test sind die Verbindungen der Formel I sowohl krankheitsverzögernd, als auch therapeutisch wirksam. Eine signifikante krankheitsverzögernde Wirkung wurde erreicht, wenn die Substanzen jeweils einmal 24 Stunden vor der Infektion in Konzentrationen von 1 - 50 mg/kg Körpergewicht bevorzugt intraperitoneal aber auch oral verabreicht wurde.

**Tabelle 2**

Überlebensraten von Candida-infizierten $CF_1$-Mäusen nach i.p. Vorbehandlung mit den angegebenen Verbindungen

| Verb. H. Beispiel | Überlebensrate der Kontrolltiere am 14. Tag | Überlebensrate der der vorbehandelten Tiere am 14. Tag |
|---|---|---|
| 11 | 1/10 | 4/10 |
| 40 | 1/10 | 4/10 |
| 42 | 0/10 | 4/10 |
| 45 | 0/10 | 6/10 |
| 46 | 0/10 | 4/10 |

**Tabelle 2 (Fortsetzung)**

| Verb.H. Beispiel | Überlebensrate der Kontrolltiere am 14. Tag | Überlebensrate der unbehandelten Tiere am 14. Tag |
|---|---|---|
| 53 | 0/10 | 4/10 |
| 54 | 0/10 | 4/10 |
| 55 | 0/10 | 6/10 |

Bei behandelten Tieren wurde eine statistische signifikante Verlängerung der Überlebenszeit im Vergleich zu den unbehandelten Kontrollen beobachtet. Etwa 50 % der behandelten Tiere überlebten einen Beobachtungszeitraum von 14 Tagen verglichen mit etwa 10 % unbehandelten Kontrolltieren.

Eine therapeutische Wirkung wurde erreicht, wenn die Tiere beginnend mit dem Tag der Infektion einmal täglich über 3 Tage mit jeweils 1 - 30 mg/kg Körpergewicht des Präparats oral oder intraperitoneal behandelt wurden.

In den behandelten Tiergruppen überlebten bis zum 14. Tag post infectionem etwa 60 % der Tiere, verglichen mit 20 % der unbehandelten Kontrolltiere.

Die prophylaktische und therapeutische Wirksamkeit der Verbindungen der Formel I im Tiermodell der Mäusecandidose läßt vermuten, daß die Substanz eine breite abwehrstimulierende Wirkung hat, die dazu führt, daß nicht nur Hefeinfektionen, sondern generell mikrobielle Erreger bei Tier und Mensch besser kontrolliert werden. Von daher scheint es gerechtfertigt, die Substanz als antiinfektiv wirksam zu bezeichnen, zumal keine Hinweise auf antifungische Aktivität der Substanz bestehen.

Die erfindungsgemäßen Verbindungen können allein als Prophylaktikum zur Abwehr bestehender Infektionen oder in Kombination mit einer antibiotischen Therapie zur Steigerung der therapeutischen Wirkung von Antibiotika und Chemotherapeutika (z.B. Penecilline, Cephalosporine Aminoglykoside etc.) bei infizierten Menschen und Tieren verwendet werden.

Es wurde gefunden, daß Infektionen der Maus mit pathogenen Keimen, die innerhalb von 24 - 48 Stunden zum Tod der Versuchstiere führen und eine prophylaktische Behandlung - bevorzugt intraperitoneal - mit 1-80 mg/kg der erfindungsgemäßen Verbindungen der Formel I therapiert werden können. Dies trifft für eine ganze Reihe grampositiver (z.B. Staphylokokken) und gramnegativer (z.B. E.coli, Klebsiella, Proteus, Pseudomonas) Krankheitserreger zu. Diese Aufzählung ist beispielhaft und keineswegs beschränkend aufzufassen. So überleben z.B. Mäuse die mit dem pathogenen Stamm Klebsiella 63 infiziert worden waren nach Behandlung (z.B. 18 Stunden vor Infektion) mit 20 mg/kg der erfindungsgemäßen Verbindung des Beispiels 14 zu 80-90 % diese Infektion während von den unbehandelten Kontrolltieren nur 0-30 % überlebten.

Ähnliche Ergebnisse ergaben auch die Verbindungen gemäß Beispiel 11, 16, 30, 34, 40, 46, 47, 50, 55.

In einem weiteren Versuch konnte gezeigt werden, daß die therapeutische Wirksamkeit von Antibiotika durch die erfindungsgemäßen Verbindungen gesteigert werden kann (Tabelle 3). So wurden Mäuse mit dem Stamm Pseudomonas W. infiziert. Diese Infektion führt bei den meisten Kontrolltieren innerhalb 24 Stunden zum Tode. Eine weitere Gruppe wurde mit 4 mg/kg Sisomicin 30 Stunden post infectionem behandelt. Es konnte gezeigt werden, daß in der Versuchsgruppe die mit 20 mg/kg der erfindungsgemäßen Verbindung des Beispiels 14 behandelt worden war, die therapeutische Wirksamkeit des Sisomicins entscheidend verbessert werden konnte.

**Tabelle 3**

Anzahl überlebende Tiere (prozent) bei Behandlung mit der Verbindung des Beispiels 14 in Verbindung mit Sisomicin bei der Therapie einer Infektion mit Pseudomonas W. bei $CF_1$-Mäusen.

| Präparat | % Überlebende Tiere am | | |
|---|---|---|---|
| | 1. | 3. | 5. Tag p. Inf. |
| 1) Verb. gem. Bsp. 14<br>20 mg/kg | 90 | 50 | 40 |
| 2) Sisomicin<br>4 mg/kg | 50 | 30 | 30 |
| 1) Verb.Bsp.14 /20 mg/kg<br>+<br>2) Sisomicin/4 mg/kg | 100 | 80 | 80 |
| Infektionskontrolle | 25 | 15 | 15 |

1) 18 Stunden vor Infektion intraperitonal
2) 30' p. Inf. sc.

Ähnliche Ergebnisse wurden auch gefunden bei den Verbindungen gemäß Beispiel 11, 16, 30, 34, 40, 46, 47, 50, 55.

Die pharmazeutischen Präparate der vorliegenden Erfindung sind vorzugsweise Tabletten oder Gelatinekapseln, welche die Wirkstoffe zusammen mit Verdünnungsmitteln, z.B. Laktose, Dextrose, Saccharose, Mannit, Sorbit, Cellulose und/ oder Schmiermitteln, z.B. Kieselerde, Talk, Stearinsäure oder Salzen davon, wie Magnesium- oder Calciumstearat, und/ oder Polyethylenglykol, enthalten. Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht, Sprengmittel, z.B. Stärken, Agar, Alginsäure oder ein Salz davon, wie Natriumalginat und/oder Brausemischungen, oder Adsorptionsmittel, Farbstoffe, Geschmackstoffe und Süssmittel. Injizierbare Präparate sind vorzugsweise isotonische wäßrige Lösungen oder Suspensionen. Suppositorien, Salben oder Cremen sind in erster .inie Fettemulsionen oder -suspensionen. Die pharmazeutischen Präparate können sterilisiert sein und/o( er Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1 % bis etwa 75 %, insbesondere von etwa 1 % bis 50 % der genannten Aktivstoffe.

Die oral applizierten Präparate der vorliegenden Erfindung können auch mit einem gegen Magensaft beständigen Überzug versehen werden.

Die erfindungsgemäßen Verbindungen können als abwehrsteigernde und imnunpotenzierende Mittel zur Behandlung von chronischen und akuten Infektionen (z.B. bakterielle, virale und parasitäre) und malignen Tumoren verwendet werden. Sie können ebenfalls als Adjuvantien bei der Vakzinierung, bei der Stimulierung von Phagocytose und bei der Behandlung von Dysregulationen des Abwehr- und Immunsystems verwendet werden.

Die nachfolgenden Beispiele erläutern die vorstehend beschriebene Erfindung, sie sollen jedoch diese in ihrem Umfange in keiner Weise einschränken.

Die Erfindung betrifft generell auch die Salze der Verbindungen I mit irgendwelchen anderen salzbildenden Gruppen, z.B. freien Carboxylgruppen, in erster Linie pharmazeutisch verwendbare, nicht toxische Salze, z.B. Metalloder Ammoniumsalze.

**Beispiele**

Die Dünnschichtchromatographie (DC) erfolgte auf Kieselgel-DC-Fertigplatten (E. Merck, Darmstadt) und die präparativen Trennungen mit Kieselgel 60 (Merck, Darmstadt).

Laufmittelsysteme: System G $CH_2Cl_2/CH_3OH$/15 %iges Ammoniumhydroxid im Verhältnis 1/1/1, davon die Unterphase; -System E $CH_2Cl_2/CH_3$-OH 20 %iges Ammoniumhydroxid im Verhältnis 8/4/1; jeweils Volumenteile.

Die Beispiele 1, 2, 17, 19, 33, 35, 38, 41, 44, 48 und 49 beschreiben die Herstellung von Zwischenprodukten, die nicht beansprucht werden.

**Beispiel 1**: N-Benzyl-β-D-glucopyranosylamin

50 g D-Glucose wurden in 1000 ml heißem Ethanol gelöst und nach Zugabe von 89 g Benzylamin für 48 Stunden bei Raumtemperatur belassen. Dann wurde mit Eis gekühlt und das Produkt mit Petrolether gefällt. Es wurde abgesaugt, mit Ether gewaschen und im Vakuum getrocknet.

$^1$H-NMR in $CD_3OD$: $\delta = 7{,}33$ breites Singulett Phenyl-H.

**Beispiel 2**: N-Dodecyl-β-D-glucopyranosylamin

18 g Glucose wurden in 50 ml Ethanol bei 70°C gerührt, dann fügte man 18,5 g Dodecylamin zu, heizte noch bis zur klaren Lösung, ließ auf Raumtemperatur abkühlen und saugte nach 20 Stunden von den ausgefallenen Kristallen ab. Man wusch mit Ethanol und Ether und trocknete im Vakuum. Ausbeute = 24 g.

Elementaranalyse: ($C_{18}H_{37}NO_5$ = 347):
ber. C = 62,2 %, H = 10,6 %, N = 4,0 %
gef. C = 62,2 %, H = 10,6 %, N = 4,2 %

**Beispiel 3**: N-Clucopyranosyl-N-propyl-ölsäureamid

11 g N-propyl-D-glucopyranosylamin wurden in 90 ml Tetrahydrofuran (THF) mit 21 g Soda gerührt, dann tropfte man 1 Äquivalent Ölsäurechlorid in 20 ml THF langsam unter Kühlen dazu. Nach vollständiger N-Acylierung (Kontrolle durch DC im Laufmittelsystem $CH_2Cl_2/CH_3OH$ = 13:1) wurde vom Niederschlag abgesaugt, mit THF nachgewaschen, die Filtrate im Vakuum eingedampft und der erhaltene Sirup an Kieselgel zur Nachreinigung chromatographiert. Entwicklung der Säule mit $CH_2Cl_2/CH_3OH$ = 15:1.

Die Fraktionen, die die Titelverbindung rein enthalten, wurden vereinigt. Das Lösungsmittel wurde im Vakuum entfernt. Ausbeute: 3,3 g.

Rf.-Wert: 0,34 in $CH_2Cl_2/CH_3OH$ = 15:1
$[\alpha]^{20}_D = +7{,}5°$ (c = 1,0 $CH_2Cl_2$)

**Beispiel 4**: N-Glucopyranosyl-N-hexyl-ölsäureamid

Die Herstellung erfolgte ausgehend von N-Hexyl-D-glucopyranosylamin wie im Beispiel 3 beschrieben. Säulenchromatographie mit $CH_2Cl_2/CH_3OH$ = 13:1.

Ausbeute: 9,2 g Reinprodukt.
Rf.-Wert = 0,38 in $CH_2Cl_2/CH_3OH$ = 13:1
$[\alpha]^{20}_D = +5{,}8°C$ (c = 0,94 in $CH_2Cl_2$)

**Beispiel 5**:. N-Glucopyranosyl-N-(n-3,3,3-trifluor-propyl)-ölsäureamid

3,6 g Glucose und 0,8 ml 0,5 N Salzsäure und 4,6 g n-3,3,3-Trifluorpropylamin wurden für 25 Minuten auf 75° C unter Rühren erhitzt. Nach Abkühlen kristallisierte das N-Glucosid aus, wurde mit Ether gewaschen und im Vakuum getrocknet.
Ausbeute: 4,1 g.
Die N-Acylierung mit Ölsäurechlorid erfolgte analog Beispiel 3. Säulenchromatographie mit $CH_2Cl_2/CH_3OH = 15:1$.
Ausbeute: 2,7 g.
$[\alpha]^{20}_D = +7{,}6°$ (c = 1,0 in $CH_2Cl_2$)

**Beispiel 6**: N-(2-Ethyl-hexyl)-N-glucopyranosyl-ölsäureamid

Die Umsetzung von Glucose mit 2-Ethyl-hexylamin erfolgte analog Beispiel 5. Die N-Acylierung mit Ölsäurechlorid wurde analog Beispiel 3 durchgeführt. Säulenchromatographie mit $CH_2Cl_2/CH_3OH = 15:1$.
Rf.-Wert der Titelverbindung: 0,44 in $CH_2Cl_2/CH_3OH = 15/1$.

**Beispiel 7**: N-(3-Butoxy-propyl)-N-glucopyranosyl-öl-

säureamid
Herstellung des N-Glycosids und N-Acylierung wie bei Beispiel 5 bzw. Beispiel 3 beschrieben.
Rf.-Wert: 0,29 Laufmittelsystem $CH_2Cl_2/CH_3OH = 10/1$.

**Beispiel 8**: N-Dodecyl-N-glucopyranosyl-stearinsäureamid
100 g N-Dodecyl-β-D-glucopyranosylamin aus Beispiel 2 wurden in 765 ml THF gelöst und in Gegenwart von 32 g Triethylamin unter Kühlen tropfenweise mit 80 g Stearinsäurechlorid versetzt.
Zur Aufarbeitung wurde filtriert und das Lösungsmittel im Vakuum entfernt.
Ebenso wurde hergestellt N-Dodecyl-N-glucopyranosyl-ölsäureamid.

**Beispiel 9**: N-Decyl-N-glucopyranosyl-ölsäureamid

18 g D-Glucose und 50 ml Ethanol wurden bei 70° C mit 15,7 g Decylamin bis zur klaren Lösung gerührt. Dann ließ man auf Raumtemperatur abkühlen, saugte nach 4 Stunden die Kristalle ab und wusch mit Ethanol und Ether nach. Ausbeute: 20 g.
Diese wurden in 166 ml THF mit 22,6 g Soda gerührt. Dann tropfte man langsam 19 g Ölsäurechlorid in 20 ml THF bei 25° C zu. Nach einer weiteren Stunde wurde abgesaugt, das Filtrat im Vakuum zum Sirup eingedampft und das Rohprodukt säulenchromatographisch an Kieselgel mit dem Elutionsmittel $CH_2Cl_2/CH_3OH = 13/1$ gereinigt.
Rf.-Wert der Titelverbindung =0,53 in $CH_2Cl_2/CH_3OH = 13/2$.

**Beispiel 10**: N-Glucopyranosyl-N-tetradecyl-Ölsäureamid

Herstellung analog Beispiel 9.
Säulenchromatographie mit dem Elutionsmittel $CH_2Cl/_2CH_3OH = 13/1$
$[\alpha]^{20}_D = +9{,}6$ (c = 1,0 DMF)
Elementaranalyse:
ber. C = 70,3%, H = 11,3%, N = 2,16%
gef. C = 69,4%, H = 11,6%, N = 2,1%

**Beispiel 11**: N-Glucopyranosyl-N-hexadecyl-ölsäureamid

Herstellung und Reinigung analog Beispiel 9
Rf.- Wert 0,25 Laufmittel $CH_2Cl_2/CH_3OH$ = 13/1

**Beispiel 12**: N-Glucopyranosyl-N-octadecyl-ölsäureamid

90 g D-Glucose und 135 g Octadecylamin wurden in 1.000 ml Propanol - 2 und 500 ml Wasser auf 50° C unter Rühren erhitzt bis eine klare Lösung entstanden war. Dann beließ man über Nacht bei Raumtemperatur. Nun wurde das Produkt abgesaugt, mit Alkohol und Ether gewaschen, getrocknet und zuletzt aus Ethanol/THF umkristallisiert. 10 g dieses N-Octadecyl-β-D-glucopyranosylamins wurden in 80 ml THF aufgeschlämmt und nach Zusatz von 10 g Soda tropfenweise mit 7 g ölsäurechlorid in 10 ml THF versetzt. Nach quantitativer Umsetzung (DC in $CH_2Cl_2/CH_3OH$ = 13/1) wurde wie in Beispiel 12 beschrieben aufgearbeitet. Säulenreinigung und Elutionsmittel $CH_2Cl_2/CH_3OH$ = 13/1.
RF.-Wert = 0,35 Laufmittelsystem $CH_2Cl_2/CH_3OH$ = 9/1.

**Beispiel 13**: N-Glucopyranosyl-N-Octadecyl-Stearinsäureamid
Herstellung analog Beispiel 3 aus N-Octadecyl-glucopyranosylamin und Stearinsäurechlorid.
Elementaranalyse:.
ber.: C = 72,5%, H = 11,7%, N = 2,0%
gef.: C = 71,7%, H = 12,2%, N = 2,0%

**Beispiel 14**: N-Glucosyl-N-octadecyl-dodecansäureamid

Herstellung analog Beispiel 3 aus N-Octadecyl-β-D-glucopyranosylamin und Dodecansäurechlorid.
$[\alpha]^{20}_D$ = + 8° (C =1,0 Dioxan)

**Beispiel 15**: N-glucosyl-N-octadecyl-tetradecansäureamid

Herstellung analog Beispiel 3 aus N-Octadecyl-β-D-glucopyranosylamin und Tetradecansäurechlorid
$[\alpha]^{20}_D$ = + 9,5° (C=1,0 DMF)
Elementaranalyse:
ber.: C = 71,8%, H = 11,7%, N = 2,1%
gef.: C =-71,3%, H = 11,9%, N = 1,9%

**Beispiel 16**: N-(2-Acethmido-2-desoxy-D-glucopyranosyl)-

N-octadecyl-Ölsäureamid
15 g N-Acetyl-D-glucosamin und 18,8 g Octadecylamin wurden für 3 Stunden in 50 ml Ethanol auf 80° C unter Rühren erhitzt. Dann wurde heiß vom Ungelösten abfiltriert, das Filtrat abgekühlt, das ausgefallene Produkt abgesaugt und mit Ethanol und Ether gewaschen 2,2 g des so erhaltenen 2-Acetamido-2-desoxy-N-octadecyl-glucopyranosylamins wurden in 17 ml THF mit 2 g Soda gerührt. Dann versetzte man tropfenweise mit 1,45 g Ölsäurechlorid in 5 ml THF.
Aufarbeitung wie in Beispiel 3 beschrieben.
Säulenchromatographie mit dem Elutionsmittel $CH_2Cl_2/CH_3OH$ =20/1.
$[\alpha]^{20}_D$ = + 9,2° (c = 0,56 $CH_3OH$)
Elementaranalyse:
ber.: C = 2,8%, H = 11,7%, N = 3,8%
gef.: C = 72,9%, H = 12,5%, N =3,3%

**Beispiel 17**: N-Octadecyl-L-rhamnopyranosylamin

9 g L-Rhamnose und 13,5 g Stearylamin wurden in 100 ml Propanol - 2 und 50 ml Wesser bei 50°C solange gerührt, bis eine klare Lösung entstanden war. Nach 50 Stunden bei Raumtemperatur wurden die Kristalle abgesaugt, mit Ethanol und Ether gewaschen und im Vakuum getrocknet. Ausbeute: 17,4 g

**Beispiel 18**: N-Octadecyl-N-rhamnopyranosyl-ölsäureamid

7 g der Verbindung aus Beispiel 17 wurden wie in Beispiel 3 beschrieben mit Ölsäurechlorid acyliert Säulentrennung in $CH_2Cl_2/CH_3OH$ = 13/1
Elementaranalyse:
ber.: C = 74,4%, H = 11,9%, N = 2,04%
gef.: C = 74,3%, H = 12,0%, N = 2,1%

**Beispiel 19**: N-Octadecyl-L-fucopyranosylamin

3,26 g L-Fucose und 5,38 g Stearylamin wurden in 20 ml Ethanol auf 70°C unter Rühren erhitzt, bis eine klare Lösung entstanden war. Man ließ abkühlen, saugte den Feststoff nach beendeter Kristallisation ab und wusch ihn mit Ethanol und Ether.
Ausbeute nach Trocknen in Vakuum: 4,4 g.

**Beispiel 20**: N-Fucopyranosyl-N-octadecyl-ölsäureamid

2,9 g der Verbindung aus Beispiel 19 wurden wie in Beispiel 3 beschrieben mit Ölsäurechlorid acyliert. Säulenchromatographie mit dem Elutionsmittel $CH_2Cl_2/CH_3OH$ = 15/1.
Ausbeute an Reinprodukt: 1,9 g
RF-Wert = 0,44 Laufmittelsystem wie bei Säulenchromatographie.

**Beispiel 21**: N-β,D-Arabinopyranosyl-N-octddecyl-öl-

säureamid
7 g N-Octadecyl-β,D-arabinopyranosylamin wurden wie in Beispiel 3 beschrieben mit ölsäurechlorid acyliert. Säulenchromatographie mit Elutionsmittel $CH_2Cl_2/CH_3OH$ = 20/1.
Ausbeute an Reinprodukt: 2,3 g
RF-Wert = 0,57 Laufmittel $CH_2Cl_2/CH_3OH$ = 15/1
$[\alpha]^{20}_D = +20°$ (c = 1,03 $CH_2CC_2$)

**Beispiel 22**: N-β,D-Maltosyl-N-octadecyl-ölsäureamid

3,04 g N-Octadecyl-β-D-maltosylamin wurden wie in Beispiel 3 beschrieben mit Ölsäurechlorid acyliert.
Säulenchromatographie in $CH_2Cl_2/CH_3OH$ = 10/1.
RF.-Wert: 0,24 Laufmittel $CH_2Cl_2/CH_3OH$ =8,1
$[\alpha]^{20}_D = +22°C$ (c = 0,5 $CH_3OH$)

**Beispiel 23**: N-(4-Azido-4-desoxy-D-glucopyranosyl)-N-

octadecyl-dodecansäureamid
3,09 g 4-Azido-4-desoxy-D-glucose wurden in 30 ml Isopropanol und 15 ml Wasser gelöst und nach Zugabe von 4,05 g Octadecylamin auf 50°C erwärmt.
Die entstandene Lösung wurde über Nacht bei Raumtemperatur stehengelassen. Der entstandene Feststoff wurde abfiltriert, mit wenig Ethanol und Ether nachgewaschen und getrocknet.
2,3 g dieses produktes wurden in 10 ml THF gelöst, mit 3 g Natriumcarbonat und 1,2 g Dodecansäurechlorid, gelöst in 15 ml THF, versetzt. Nach quantitativer Umsetzung wurde wie bei Beispiel 9 beschrieben

aufgearbeitet.
Rf.-Wert: 0,27 in $CH_2Cl_2/CH_3OH$ = 4 : 1 (V/V)

**Beispiel 24**: N-(4-Acetamido-4-desoxy)-D-glucopyranosyl-

N-octadecyl-ölsäureamid

3 g der Verbindung aus Beispiel 23 wurden in 30 ml Dioxan/ Methanol = 2/1 und 3 ml Acetanhydrid in Gegenwart von 1,0 g Palladiumkohle (5%) bei Normaldruck hydriert. Nach Beendigung der Reaktion (Laufmittelsystem $CH_2Cl_2/CH_3OH$ = 3/1 wurde vom Katalysator abfiltriert und im Vakuum eingeengt.
Rf.-Wert: 0,18 ($CH_2Cl_2$/MeOH, 10:1 V/V)

**Beispiel 25**: N-(6-Desoxy-6-fluoro-D-glucopyranosyl)-N-

octadecyl-ölsäureamid

18,2 g 6-Desoxy-6-fluor-D-glucose und 13,5 g Octadecylamin und 7 g Ölsäurechlorid wurden wie in Beispiel 12 beschrieben umgesetzt und aufgearbeitet.
Rf.-Wert: 0,30 in $CH_2Cl_2/CH_3OH$ = 9/1

**Beispiel 26**: N-(methyl-D-glucopyranosyl)uronato-N-octa-

decyl-ölsäureamid

15 g D-Glucuronolacton wurden in 150 ml abs. Methanol gelöst und mit 3 ml 1 N Natriummethanolat-Lösung eine halbe Stunde bei Raumtemperatur stehengelassen. Anschließend wurde mit saurem Ionenaustauscher neutralisiert und eingedampft. Der entstandene Glucuronsäuremethylester wurde wie bei Beispiel 12 beschrieben zur Titelverbindung umgesetzt und aufgearbeitet.
Rf-Wert: 0,32 ($CH_2Cl_2/CH_3OH$ = 9:1, V/V)

**Beispiel 27**: N-(Glucuronopyranosyl)-N-octadecyl-ölsäure-

amid

2 g der in Beispiel 26 beschriebenen Verbindung wurden in 10 ml Dioxan gelöst und nach Zugabe von 5 ml 1 N Natronlauge 2 h am Rückfluß erhitzt. Nach dem Abkühlen wurde mit verd. Salzsäure neutralisiert, im Vakuum eingeengt und der Rückstand mit 20 ml Methanol/Dioxan = 1/1 gerührt. Anschließend wurde filtriert und das Filtrat zum Sirup eingeengt.
Rf.-Wert: 0,13 ($CH_2Cl_2/CH_3OH$ = 7:1, V/V)

**Beispiel 28**: N-(4-Amino-4-desoxy-D-glucopyranosyl)-N-octa-

decyl-laurinsäureamid

3 g der Verbindung aus Beispiel 27 wurden in 30 ml Dioxan/ Methanol 2/1 in Gegenwart von 1,0 g Palladiumkohle (5%) hydriert. Nach Beendigung der Reaktion wurde vom Katalysator abfiltriert und i.Vac. eingeengt.
Rf.-Wert: 0,39 $CH_2Cl_2$/MeOH 5:1

**Beispiel 29**: N-(4-laurinamido-4-desoxy-D-glucopyranosyl)-

N-octadecy-laurinsäureamid

4,00 g in Beispiel 28 beschriebenen Verbindung wurden in 30 ml THF gelöst, mit 2,0 g Natriumcarbonat versetzt und mit 1,42 g Dodecansäurechlorid in 10 ml THF umgesetzt. Nach 30 Min. wurde mit Dichlormethan verdünnt, filtriert und das Filtrat i. Vac. eingeengt. Der Sirup wurde Säulenchromatographisch (Laufmittel Dichlormethan/Methanol = 15:1) gereinigt.
Rf.-Wert: 0,36 $CH_2Cl_2$/MeOH 10:1

**Beispiel 30**: N-Glucopyranosyl-N-octadecyl-Palmitinsäureamid

Herstellung analog Beispiel 13 aus N-Octadecyl-glucopyranosylamin und palmitinsäurechlorid.
Rf.-Wert: 0,36 $CH_2Cl_2$/MeOH 9:1

**Beispiel 31**: N-Octadecyl-N-glucopyranosyl-laurinsäureamid

Herstellung analog Beispiel 13 aus N-Octadecyl-glucopyranosylamin und Laurinsäurechlorid.
Rf-Wert: 0,35 $CH_2Cl_2$/$CH_3OH$ 9:1

**Beispiel 32**: N-Octadecyl-N-rhammopyranosyl-stearinsäureamid
Herstellung analog Beispiel 18 aus N-Octadecyl-rhammopyranosylamin und Stearinsäurechlorid.
Rf-Wert: 0,39 $CH_2Cl_2$/$CH_3OH$ 9:1

**Beispiel 33**: N-Octadecyl-(2-Amino-2-desoxy-D-glucopyranosyl)

amin-Hydrochlorid
6,45 g D-Glucosaminhydrochlorid wurden bei 60°C in 30 ml iso-Propanol und 10 ml Wasser gelöst und mit 12,1 g Stearylamin versetzt. Die entstandene klare Lösung wurde noch 10 min. weiter gerührt und dann auf Raumtemperatur abgekühlt. Das auskristalliisierte Produkt wurde abgesaugt und zunächst mit Ethanol/Wasser (5:2, v/v), dann mit Ethanol und schließlich mit Ether gewaschen. Der Rückstand wurde am Hochvakuum getrocknet.

**Beispiel 34**: N-Octadecyl-N-(2-dodecylamino-2-desoxy-D-

glucopyranosal)-dodecansäureamid
4,6 g der in Beispiel 33 beschriebenen Verbindung wurden in 120 ml Tetrahydrofuran aufgeschlämmt und mit 22,6 g Natriumcarbonat versetzt. Zu der gerührten Suspension wurden 4,2 g Dodecansäurechlorid in 20 ml Tetrahydrofuran zugetropft. Der Ansatz wurde i.Vak. eingedampft, mit 50 ml Pyridin und 25 ml Acetanhydrid acetyliert, auf Eiswasser gegossen, in Dichlormethan aufgenommen, die organische Phase wird nacheinander mit verdünnter Salzsäure, gesättigter Natriumhydrogencarbonatlösung und dann mit Wasser gewaschen, über Natriumsulfat getrocknet und i.Vak. zum Sirup eingedampft. Der erhaltene Sirup wurde säulenchromatographisch gereinigt. (Laufmittel Toluol/Essigester 10 : 1, v/v). Der erhaltene Feststoff (Schmp. 86°) wurde in abs. Methanol gelöst, mit 20 mg Natriummethoxid versetzt und 20 min unter Rückfluß erhitzt. Nach Beendigung der Reaktion wurde mit saurem Ionenaustauscher neutralisiert und i.Vak. eingedampft.
Schmelzpunkt: 78°C, Rf.-Wert: 0,64 in $CH_2Cl_2$/MeOH = 10/1 (v/v)

**Beispiel 35**: N-propyl-(2-amino-2-desoxy-D-glucopyranosyl)

amin-Hydrochlorid
21,5 g Glucosaminhydrochlorid wurden in 17,7 g n-Propylamin aufgeschlämmt und auf 70° erwärmt, bis eine klare Lösung entstand. Beim Abkühlen auf Rt. fiel das Produkt aus.

**Beispiel 36**: N-propyl-N-(2-ölsäureamido-2-desoxy-D-Gluco-

pyranosyl)ölsäureamid
5,1 g der in Beispiel 35 beschriebenen Verbindung wurden in 100 ml Tetrahydrofuran aufgeschlämmt, mit 12,7 g Natriumcarbonat versetzt. Anschließend wurden 12 g Ölsäurechlorid in 20 ml Tetrahydrofuran zugetropft. Nach Beendigung der Reaktion wurde der Ansatz mit 50 ml Dichlormethan verdünnt, vom Natriumsalz abfiltriert, mit Wasser gewaschen, getrocknet über Natriumsulfat und i.Vak. eingedampft. Das erhaltene Sirup wurde säulenchromatographisch gereinigt (Laufmittel Dichlormethan/Methanol 15/1, v/v).

Rf.-Wert: 0,37 in $CH_2Cl_2$/MeOH 10:1
$\alpha_D$ = 17,9° (c = 1,02 in Dichlormethan)

**Beispiel 37**:. N-Glucopyranosyl-N-tetradecyl-stearinsäure-

amid
Herstellung analog Beispiel 9 aus N-Tetradecyl-glucopyranosylamin Sterinsäurechlorid.
Rf.-Wert: 0,25 in Toluol/Aceton 1:1

**Beispiel 38**: N-Dodecyl-N-(2-amino-2-desoxy-glucopyranosyl)-

amin Hydrochlorid
46 g Dodecylamin wurden bei 60° aufgeschmolzen und unter Rühren mit 31 g Glucosaminhydrochlorid versetzt. Nach Abkühlen auf Rt. fiel das produkt aus. Der Feststoff wurde dreimal mit Ether aufgerührt und abgesaugt und anschließend im Hochvakuum getrocknet.

**Beispiel 39**: N-Dodecyl-N-(2-stearylamido-2-desoxy-D-gluco-

pyranosyl)-stearinsäureamid
5 g der in Beispiel 38 beschriebenen Verbindung wurden in 100 ml Tetrahydrofuran aufgeschlämmt, mit 8,5 g Natriumcarbonat und 8 g Stearinsäurechlorid in 20 ml Tetrahydrofuran versetzt. Nach Beendigung der Reaktion wurde wie in Beispiel 36 beschrieben aufgearbeitet. Der erhaltene Rohsirup wurde aus Essigester kristallisiert.
Schmp. 67°; Rf.-Wert 0,42 in $CH_2Cl_2$/MeOH 10/1

**Beispiel 40**: N-Dodecyl-N-(2-laurinsäureamido-2-desoxy-D-

glucopyranosyl)-laurinsäureamid
5 g der in Beispiel 38 beschriebenen Verbindung wurden wie bei Beispiel 39 beschrieben mit Laurinsäurechlorid umgesetzt.
Schmp. 67°; Rf.-Wert 0,42 in $CH_2Cl_2$/MeOH 10/1

**Beispiel 41**: N-Octadecyl-N-(galactopyranosyl)-amin

60 g D-Galactose wurden in 330 ml Isopropanol und 170 ml Wasser suspendiert und auf 50° erwärmt. Nach Zugabe von 90 g Stearylamin wurde so lange gerührt, bis alles Amin in Lösung gegangen war. Beim Erkalten kristallisierte das Glycosylamin aus. Der Feststoff wurde abgesaugt, nacheinander mit Ethanol und mit Ether gewaschen und i.Vak. getrocknet.

**Beispiel 42**: N-Octadecyl-N-(d-galactopyranosyl)-laurinsäure-

amid
Herstellung aus 8,4 g der in Beispiel 41 beschriebenen Verbindung und 4,4 g Dodecansäurechlorid analog Beispiel 8.
Rf.-Wert: 0,22 in Toluol-n-Propanol 4/1 (v/v)
$\alpha_D$ 11,4 (c = 0,93 in Dichlormethan)

**Beispiel 43**: N-Tetradecyl-N-(D-galactopyranosyl)-ölsäure-

amid
Aus 30 g D-Galactose und 53 g Tetradecylamin wurde wie bei Beispiel 41 beschrieben N-Tetradecyl-N-(D-galactopyranosyl)-amin hergestellt. Das Galactosylamin wurde nach dem in Beispiel 8 beschriebenen

Verfahren mit Ölsäurechlorid umgesetzt.
Rf.-Wert: 0,26 in Toluol/n-Propanol 4/1 (v/v)
$\alpha = 11^\circ$ (c = 1,0 in Dichlormethan)

**Beispiel 44**: N-Octadecyl-N-mannopyranosyl-amin

20 g D-Mannose und 45 g Stearylamin wurden wie in Beispiel 41 beschrieben zum Clycosylamin umgesetzt.

**Beispiel 45**: N-Octadecyl-N-(D-mannopyranosyl)-laurinsäure-

amid
8,6 g der bei Beispiel 44 beschriebenen Verbindung wurden mit 4,4 g Dodecansäurechlorid wie bei Beispiel 8 beschrieben umgesetzt.
Rf.-Wert: 0,25 in Toluol/n-propanol 4/1 (v/v)
$\alpha_D = 11{,}3^\circ$ (c = 1,13 in Dichlormethan)

**Beispiel 46**: N-Octadecyl-N-(D-mannopyranosyl)-tetradecan-

säureamid
Herstellung aus der unter Beispiel 44 beschriebenen Verbindung und Tetradecansäurechlorid analog zu Beispiel 8.
Rf.-Wert: 0,26 in Toluol/n-propanol 4/1 (v/v)
$\alpha = 9{,}9^\circ$ (c = 1,0 in Dichlormethan)

**Beispiel 47**: N-Tetradecyl-N-(D-mannopyranosyl)-ölsäureamid

20 g D-Mannose und 35 g Tetradecylamin wurden wie bei Beispiel 41 beschrieben zum N-Tetradecylmannopyranosylamin umgesetzt. In einem zweiten Reaktionsschritt wurde das Glycosylamin (7,5 g) mit 6,0 g Ölsäurechlorid wie im Beispiel 8 beschrieben zum Glycosylamid umgesetzt.
Rf.-Wert: 0,29 in Toluol/n-Propanol 4/1 (v/v)
$\alpha_D = 10{,}8^\circ$ (c = 1 in Tetrahydrofuran)

**Beispiel 48**: 2-Dodecylamino-2-desoxy-D-glucopyranose

55 g Dodecansäurechlorid wurden in 170 ml Tetrahydrofuran gelöst und unter starkem Rühren zu einer Lösung von 54 g D-Glucosamin-hydrochlorid in 330 ml wäßriger Natriumcarbonatlösung (20 %) getropft. Nach Beendigung der Säurechloridzugabe wurde noch eine Stunde weiter gerührt, dann wurde der Ansatz mit 500 ml Wasser versetzt und der Feststoff abgesaugt und mit Wasser gewaschen. Der Rückstand wurde aus iso-Propanol/Wasser 10/1 (v/v) umkristallisiert und im Hochvakuum getrocknet.

**Beispiel 49**: N-Dodecyl-N-(2-dodecylamido-2-desoxy-D-

glucopyranosyl)-amin
15 g der in Beispiel 48 beschriebenen Verbindung wurden mit 45 g Dodecylamin und 75 ml Ethanol versetzt und unter Rühren auf 70° erwärmt. Nachdem sich eine klare Lösung gebildet hatte, wurde auf Raumtemperatur abgekühlt und über Nacht kristallisiert. Der ausgefallene Feststoff wurde abgesaugt, einmal mit Ethanol und dreimal mit Ether gewaschen und i.Vak. getrocknet.

**Beispiel 50**: N-Dodecyl-N-(2-dodecylamido-2-desoxy-D-glucopyranosyl)

-stearinsäureamid

4 g der in Beispiel 49 beschriebenen Verbindung werden in 100 ml Tetrahydrofuran gelöst und mit 4,8 g Natriumcarbonat versetzt. Zu dieser Suspension werden unter Rühren 3,45 g Stearinsäurechlorid in 20 ml Tetrahydrofuran gelöst zugetropft. Es wurde 30 min. weitergerührt, dann wurde mit 50 ml Dichlormethan verdünnt und vom Feststoff abgesaugt. Der Rückstand wurde mit Dichlormethan gewaschen. Die organischen Lösungsmittelphasen wurden vereinigt und i.Vak. eingeengt. Der erhaltene Sirup wurde chromatographisch gereinigt. (Laufmittel: Dichlormethan/Methanol 20/1 (v/v).

Rf.-Wert: 0,55 in Dichlormethan/Methanol 10/1 (v/v)

$\alpha = 15{,}8^{\circ}$ (c = 1,05 in Dichlormethan)

**Beispiel 51**: N-Dodecyl-N-(2-acetamido-2-desoxy-D-glucopyranosyl)

-tetradecansäureamid

26 g N-Acetylglucosamin wurden in 100 ml Ethanol und 60 ml Wasser gelöst und auf 60° erwärmt. Es wurden 37 g Dodecylamin hinzugegeben und bis zur Einstellung einer klaren Lösung gerührt. Nach dem Abkühlen auf Raumtemperatur kristallisierte das Clycosylamin aus. Der Kristallbrei wurde abgesaugt, mit Ethanol und dann mit Ether gewaschen und i.Vak. getrocknet. 3 g des Feststoffes wurden in 50 ml Tetrahydrofuran aufgeschlämmt, mit 3,3 g Natriumcarbonat versetzt und mit 1,9 g Tetradecansäurechlorid in 10 ml Tetrahydrofuran versetzt. Nach Beendigung der Reaktion wurde mit 30 ml Dichlormethan verdünnt, filtriert und das Filtrat i.Vak. eingedampft. Der erhaltene Sirup wurde chromatographisch gereinigt (Laufmittel Dichlormethan/Methanol 20/1, (v/v)).

Rf.-Wert: 0,21 in Dichlormethan/Methanol 10/1 (v/v)

**Beispiel 52**: N-Dodecyl-N-(2-acetamido-2-desoxy-D-glucopyranosyl)

-stearinsäureamid

3 g N-(2-Acetamido-2-desoxy)-dodecylamin, dessen Darstellung in Beispiel 51 beschrieben ist, wurden wie in Beispiel 51 beschrieben mit Stearinsäurechlorid umgesetzt.

Rf.-Wert: 0,23 in Dichlormethan/Methanol 10/1 (v/v)

**Beispiel 53**: N-Octadecyl-N-(2-acetamido-2-desoxy-D-glucopyranosyl)

-tetradecansäureamid

Herstellung analog Beispiel 16 aus N-Acetylglucosamin, Stearylamin und Tetradecansäurechlorid.

Rf.-Wert: 0,25 in Toluol/iso-Propanol 4/1 (v/v)

$\alpha = 16{,}9^{\circ}$ (c = 1 in Tetrahydrofuran)

**Beispiel 54**: N-Dodecyl-N-(D-mannopyranosyl)-stearinsäure-

amid

Herstellung aus D-Mannose, Dodecylamin und Stearinsäurechlorid analog Beispiel 8.

Rf.-Wert: 0,28 in Toluol/n-Propanol 4/1 (v/v)

$\alpha = 11{,}4^{\circ}$ (c = 1 in Tetrahydrofuran)

**Beispiel 55**: N-Dodecyl-N-(D-galactopyranosyl)-stearin-

säureamid

Herstellung aus D-Galactose, Dodecylamin und Stearinsäurechlorid analog Beispiel 8.

Rf.-Wert: 0,28 in Toluol/n-Propanol 4/1 (v/v)

$\alpha = 4{,}4^{\circ}$ (c = 1 in Dichlormethan)

**Patentansprüche:**

1. Verbindungen der allgemeinen Formel (I)

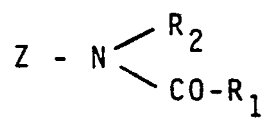

(I)

worin

Z einen über das anomere Kohlenstoffatom gebundenen Monosaccharidrest oder Maltoserest,

$R_1$ einen gegebenenfalls substituierten geradkettigen oder verzweigten, gesättigten Alkylrest mit 9 bis 21 C-Atomen oder einen ein- oder zweifach ungesättigten Alkenylrest mit 7 bis 21 C-Atomen und

$R_2$ einen geradkettigen oder verzweigten, gesättigten oder ein- oder mehrfach ungesättigten, gegebenenfalls durch bis zu 5 Sauerstoffatome enthaltende Gruppen oder Halogenatome substituierten Alkyloder Aralkylrest mit bis zu 30 C-Atomen oder einen Alkoxyalkylrest oder (Alkoxy-alkoxy)-alkylrest mit bis zu 30 O- und C-Atomen bedeuten.

2. Verbindungen nach Anspruch 1, dadurch gekennzeichnet, daß $R_2$ einen Alkyl oder Alkenylrest mit bis zu 20 C-Atomen darstellt.

3. Verbindungen nach den Ansprüchen 1 - 2, dadurch gekennzeichnet, daß Z einen Monosaccharidrest oder Maltoserest darstellt, der durch eine Acylamidgruppe substituiert ist.

4. N-Octadecyl-N-D-glucopyranosyl-laurinsäureamid.

5. N-Octadecyl-N-D-glucopyranosyl-ölsäureamid.

6. Verfahren zur Herstellung der Verbindungen der Formel (I)

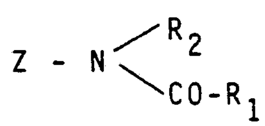

(I)

worin

Z einen über das anomere Kohlenstoffatom gebundenen Monosaccharidrest oder Maltoserest,

$R_1$ einen gegebenenfalls substituierten geradkettigen oder verzweigten, gesättigten Alkylrest mit 9 bis 21 C-Atomen oder einen ein- oder zweifach ungesättigten Alkenylrest mit 7 bis 21 C-Atomen und

$R_2$ einen geradkettigen oder verzweigten, gesättigten oder ein- oder mehrfach ungesättigten, gegebenenfalls durch bis zu 5 Sauerstoffatome enthaltende Gruppen oder Halogenatome substituierten Alkyloder Aralkylrest mit bis zu 30 C-Atomen oder einen Alkoxyalkylrest oder (Alkoxy-alkoxy)-alkylrest mit bis zu 30 O- und C-Atomen bedeuten,

dadurch gekennzeichnet, daß man ein Monosaccharid oder Maltose mit einem Amin der Formel $R_2$-$NH_2$ zu einer Verbindung

Z - NH - $R_2$

umsetzt, wobei Z und $R_2$ die oben angegebenen Bedeutungen haben, und anschließend mit einem Acylierungsmittel der Formel $R_1$-CO-X, in der $R_1$ die oben angegebene Bedeutung hat und X eine in Acylierungsreaktionen übliche Abgangsgruppe darstellt, in an sich bekannter Weise acyliert.

7. Verwendung der Verbindungen der allgemeinen Formel (I),

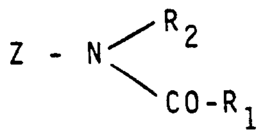

(I)

worin

Z einen über das anomere Kohlenstoffatom gebundenen Monosaccharidrest oder Maltoserest,

$R_1$ einen gegebenenfalls substituierten geradkettigen oder verzweigten, gesättigten Alkylrest mit 9 bis 21 C-Atomen oder einen ein- oder zweifach ungesättigten Alkenylrest mit 7 bis 21 C-Atomen und

$R_2$ einen geradkettigen oder verzweigten, gesättigten oder ein- oder mehrfach ungesättigten, gegebenenfalls durch bis zu 5 Sauerstoffatome enthaltende Gruppen oder Halogenatome substituierten Alkyloder Aralkylrest mit bis zu 30 C-Atomen oder einen Alkoxyalkylrest oder (Alkoxy-alkoxy)-alkylrest mit bis zu 30 O- und C-Atomen bedeuten,

zur Herstellung von Arzneimitteln, die die körpereigene Abwehr beeinflussen.

8. Verwendung der Verbindungen der allgemeinen Formel (I)

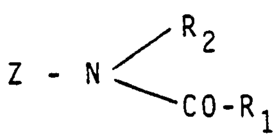

(I)

worin

Z einen über das anomere Kohlenstoffatom gebundenen Monosaccharidrest oder Maltoserest,

$R_1$ einen gegebenenfalls substituierten geradkettigen oder verzweigten, gesättigten Alkylrest mit 9 bis 21 C-Atomen oder einen ein- oder zweifach ungesättigten Alkenylrest mit 7 bis 21 C-Atomen und

$R_2$ einen geradkettigen oder verzweigten, gesättigten oder ein- oder mehrfach ungesättigten, gegebenenfalls durch bis zu 5 Sauerstoffatome enthaltende Gruppen oder Halogenatome substituierten Alkyloder Aralkylrest mit bis zu 30 C-Atomen oder einen Alkoxyalkylrest oder (Alkoxy-alkoxy)-alkylrest mit bis zu 30 O- und C-Atomen bedeuten,

zur Herstellung von Arzneimitteln, durch welche die humorale Immunität potenziert wird.

9. Verwendung der Verbindungen der allgemeinen Formel (I)

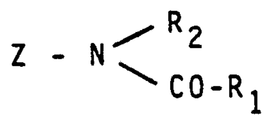

(I)

worin

Z einen über das anomere Kohlenstoffatom gebundenen Monosaccharidrest oder Maltoserest,

$R_1$ einen gegebenenfalls substituierten geradkettigen oder verzweigten, gesättigten Alkylrest mit 9 bis 21 C-Atomen oder einen ein- oder zweifach ungesättigten Alkenylrest mit 7 bis 21 C-Atomen und

$R_2$ einen geradkettigen oder verzweigten, gesättigten oder ein- oder mehrfach ungesättigten, gegebenenfalls durch bis zu 5 Sauerstoffatome enthaltende Gruppen oder Halogenatome substituierten Alkyloder Aralkylrest mit bis zu 30 C-Atomen oder einen Alkoxyalkylrest oder (Alkoxy-alkoxy)-alkylrest mit bis zu 30 O- und C-Atomen bedeuten,

zur Herstellung von Arzneimitteln, die der Stimulierung der Phagocytose dienen.

10. Verbindungen der allgemeinen Formel (I)

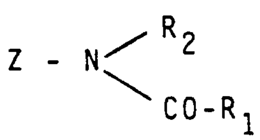

(I)

worin

Z einen über das anomere Kohlenstoffatom gebundenen Monosaccharidrest oder Maltoserest,

$R_1$ einen gegebenenfalls substituierten geradkettigen oder verzweigten, gesättigten Alkylrest mit 9 bis 21 C-Atomen oder einen ein- oder zweifach ungesättigten Alkenylrest mit 7 bis 21 C-Atomen und

$R_2$ einen geradkettigen oder verzweigten, gesättigten oder ein- oder mehrfach ungesättigten, gegebenenfalls durch bis zu 5 Sauerstoffatome enthaltende Gruppen oder Halogenatome substituierten Alkyloder Aralkylrest mit bis zu 30 C-Atomen oder einen Alkoxyalkylrest oder (Alkoxy-alkoxy)-alkylrest mit bis zu 30 O- und C-Atomen bedeuten,

zur Beeinflussung der körpereigenen Abwehr, zur Potenzierung der humoralen Immunität oder zur Stimulierung der Phagocytose des menschlichen oder tierischen Körpers.

**Claims**

1. Compounds of the general formula (I)

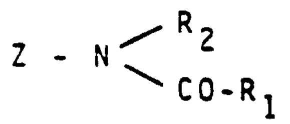

(I)

wherein

Z denotes a monosaccharide radical or a maltose radical bonded via the anomeric carbon atom,

R denotes an optionally substituted straight-chain or branched, saturated alkyl radical with 9 to 21 C atoms or a mono- or diunsatuated alkenyl radical with 7 to 21 C atoms and

$R_2$ denotes a straight-chain or branched, saturated or mono- or polyunsaturated alkyl or aralkyl radical with up to 30 C atoms which is optionally substituted by groups containing up to 5 oxygen atoms,or halogen atoms, or an alkoxyalkyl radical or (alkoxyalkoxy)-alkyl radical with up to 30 O and C atoms.

2. Compounds according to Claim 1, characterised in that $R_2$ represents an alkyl or alkenyl radical with up to 20 C atoms.

3. Compounds according to Claims 1 - 2, characterised in that Z represents a monosaccharide radical or a maltose radical which is substituted by an acylamide group.

4. N-Octadecyl-N-D-glucopyranosyl-lauric acid amide.

5. N-Octadecyl-N-D-glucopyranosyl-oleic acid amide.

6. Process for the preparation of the compounds of the formula (I)

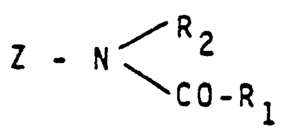

(I)

wherein

Z denotes a monosaccharide radical or a maltose radical bonded via the anomeric carbon atom,

$R_1$ denotes an optionally substituted straight-chain or branched, saturated alkyl radical with 9 to 21 C atoms or a mono- or diunsaturated alkenyl radical with 7 to 21 C atoms and

$R_2$ denotes a straight-chain or branched, saturated or mono- or polyunsaturated alkyl or aralkyl radical with up to 30 C atoms which is optionally substituted by groups containing up to 5 oxygen atoms, or halogen atoms, or an alkoxyalkyl radical or talkoxyalkoxy)-alkyl radical with up to 30 O and C atoms,

characterised in that monosaccharide or maltose is reacted with an amine of the formula $R_1$-$NH_2$ to give a compound

Z - NH - $R_2$

wherein

Z and $R_2$ have the abovementioned meanings, and the product is then acylated in a manner known per se with an acylating agent of the formula $R_1$-CO-X, in which $R_1$ has the abovementioned meaning and X reresents a leaving group customary in acylation reactions.

7. Use of the compounds of the general formula (I),

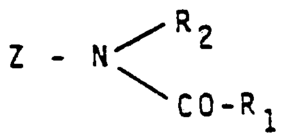

(I)

wherein

Z denotes a monosaccharide radical or a maltose radical bonded via the anomeric carbon atom,

$R_1$ denotes an optionally substituted straight-chain or branched, saturated alkyl radical with 9 to 21 C atoms or a mono- or diunsaturated alkenyl radical with 7 to 21 C atoms and

$R_2$ denotes a straight-chain or branched, saturated or mono- or polyunsaturated alkyl or aralkyl radical with up to 30 C atoms which is optionally substituted byg groups containing up to 5 oxygen atoms or halogen atoms, or an alkoxyalkyl-radical or (alkoxy-alkoxy)alkyl radical with up to 30 O and C atoms,

for the preparation of medicaments which influence the body's inherent defences.

8. Use of the compounds of the general formula (I)

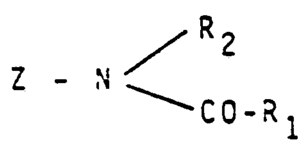

(I)

wherein

Z denotes a monosaccharide radical or a maltose radical bonded via the anomeric carbon atom,

$R_1$ denotes an optionally substituted straight-chain or branched, saturated alkyl radical with 9 to 21 C atoms or a mono- or diunsaturated alkenyl radical with 7 to 21 C atoms and

$R_2$ denotes a straight-chain or branched, saturated or mono- or polyunsaturated alkyl or aralkyl radical with up to 30 C atoms which is optionally substituted byg roups containing up to 5 oxygen atoms, or halogen atoms, or an alkoxyalkyl radical or (alkoxy-alkoxy)-alkyl radical with up to 30 O and C atoms,

for the preparation of medicaments by means of which humoral immunity is potentiated.

9. Use of the compounds of the general formula (I)

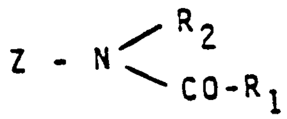

(I)

wherein
Z denotes a monosaccharide radical or a maltose radical bonded via the anomeric carbon atom,
$R_1$ denotes an optionally substituted straight-chain or branched, saturated alkyl radical with 9 to 21 C atoms or a mono- or diunsaturated alkenyl radical with-7 to 21 C atoms and
$R_2$ denotes a straight-chain or branched, saturated or mono- or polyunsaturated alkyl or aralkyl radical with up to 30 atoms which is optionally substituted by groups containing up to 5 oxygen atoms,or halogen atoms, or an alkoxyalkyl radical or (alkoxy-alkoxy)alkyl radical with up to 30 O and C atoms,
for the preparation of medicaments which serve to stimulate phagocytosis.

10. . Compounds of the general formula (I)

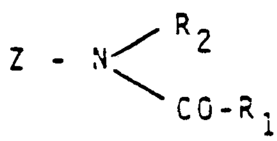

(I)

wherein
Z denotes a monosaccharide radical or a maltose radical bonded via the anomeric carbon atom,
$R_1$ denotes an optionally substituted straight-chain or branched, saturated alkyl radical with 9 to 21 C atoms or a mono- or diunsaturated alkenyl radical with 7 to 21 C atoms and
$R_2$ denotes a straight-chain or branched, saturated or mono- or polyunsaturated alkyl or aralkyl radical with up to 30 C atoms which is optionally substituted by groups containing up to 5 oxygen atoms, or halogen atoms, or an alkoxyalkyl radical or (alkoxy-alkoxy)alkyl radical with up to 30 O and C atoms,
for influencing the body's inherent defences, for potentiating humoral immunity or for stimulating phagocytosis in the human or animal body.

**Revendications**

1. Composés de formule générale (I) :

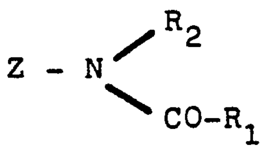

(I)

dans laquelle

Z représente un radical monosaccharide ou un radical maltose relié par l'atome de carbone anomère,

$R_1$ représente un radical alkyle saturé, à chaîne droite ou ramifiée, éventuellement substitué et contenant 9 à 21 atomes de carbone ou un radical alcényle insaturé une ou deux fois et comportant 7 à 21 atomes de carbone, et

$R_2$ représente un radical alkyle ou un radical aralkyle contenant jusqu'à 30 atomes de carbone, à chaîne droite ou ramifiée, saturé ou insaturé une ou plusieurs fois et éventuellement substitue par des atomes d'halogènes ou des groupes contenant jusqu'à 5 atomes d'oxygène, ou encore un radical alcoxyalkyle ou un radical (alcoxy-alcoxy)-alkyle comportant jusqu'à 30 atomes d'O et de C.

2. Composés selon la revendication 1, caractérisés en ce que $R_2$ represente un radical alkyle ou un radical alcényle contenant jusqu'à 20 atomes de carbone.

3. Composés selon les revendications 1-2, caractérisés en ce que Z représente un radical monosaccharide ou un radical maltose qui est substitute par un groupe acylamido.

4. Amide d'acide N-octadécyl-N-D-glucopyranosyl-laurique.

5. Amide d'acide N-octadécyl-N-D-glucopyranosyl-oléique.

6. Procédé de préparation des composés de formule (I) :

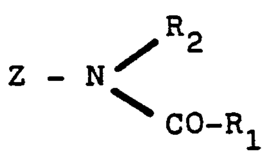

(I)

dans laquelle

Z représente un radical monosaccharide ou un radical maltose relié par l'atome de carbone anomère.

$R_1$ représente un radical alkyle saturé à chaîne droite ou ramifiée, éventuellement substitué et contenant 9 à 21 atomes de carbone ou un radical alcényle insaturé une ou deux fois et contenant 7 à 21 atomes de carbone, et

$R_2$ représente un radical alkyle ou un radical aralkyle contenant jusqu'à 30 atomes de carbone, à chaîne droite ou ramifiée, saturé ou insaturé une ou plusieurs fois et éventuellement substitué par des atomes d'halogènes ou des groupes contenant jusqu'à 5 atomes d'oxygène, ou un radical alcoxyalkyle ou un radical (alcoxy-alcoxy)-alkyle contenant jusqu'à 30 atomes d'O et de C,

caractérisé en ce qu'on fait réagir un monosaccharide ou maltose avec une amine de formule $R_1$-$NH_2$ pour obtenir un composé

Z -NH - $R_2$

Z et $R_2$ ayant les significations indiquées ci-dessus, puis on procède à une acylation de façon connue en soi avec un agent d'acylation de formule $R_1$-CO-X dans laquelle $R_1$ a la signification indiquée ci-dessus et X représente un groupe de départ intervenant habituellement dans des réactions d'acylation.

7. Utilisation des composés de formule générale (I) :

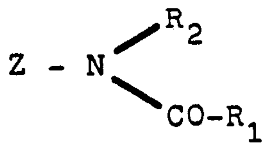

(I)

dans laquelle
Z représente un radical monosaccharide ou un radical maltose relié par l'atome de carbone anomère,
$R_1$ représente un radical alkyle saturé, à chaîne droite ou ramifiée, éventuellement substitué et contenant 9 à 21 atomes de carbone ou un radical alcényle insaturé une ou deux fois et contenant 7 à 21 atomes de carbone, et
$R_2$ représente un radical alkyle ou un radical aralkyle contenant jusqu'à 30 atomes de carbone, à chaîne droite ou ramifiée, saturé ou insaturé une ou plusieurs fois et éventuellement substitué par des atomes d'halogènes ou des groupes contenant jusqu'à 5 atomes d'oxygène, ou un radical alcoxyalkyle ou encore un radical (alcoxy-alcoxy)-alkyle contenant jusqu'à 30 atomes d'O et de C,
pour la préparation de médicaments influençant la défense propre au corps.

8. Utilisation des composés de formule générale (I) :

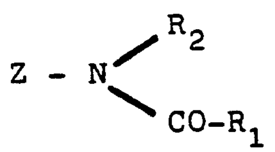

(I)

dans laquelle
Z représente un radical monosaccharide ou un radical maltose relié par l'atome de carbone anomère,
$R_1$ représente un radical alkyle saturé, à chaîne droite ou ramifiée, éventuellement substitué et contenant 9 à 21 atomes de carbone ou un radical alcényle insaturé une ou deux fois et contenant 7 à 21 atomes de carbone, et
$R_2$ represente un radical alkyle ou un radical aralkyle contenant jusqu'à 30 atomes de carbone, à chaîne droite ou ramifiée, saturé ou insaturé une ou plusieurs fois et éventuellement substitué par des atomes d'halogènes ou des groupes contenant jusqu'à 5 atomes d'oxygène, ou un radical alcoxyalkyle ou encore un radical (alcoxy-alcoxy)-alkyle contenant jusqu'à 30 atomes d'O et de C,
pour la préparation de médicaments par lesquels l'immunité humorale est potentialisée.

9. Utilisation des composés de formule générale (I) :

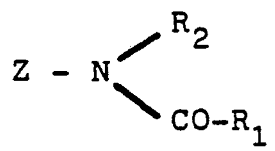 (I)

dans laquelle

Z représente un radical monosaccharide ou un radical maltose relié par l'atome de carbone anomère,

$R_1$ représente un radical alkyle saturé, à chaîne droite ou ramifiée, éventuellement substitué et comportant 9 à 21 atomes de carbone ou un radical alcényle insaturé une ou deux fois et contenant 7 à 21 atomes de carbone, et

$R_2$ représente un radical alkyle ou un radical aralkyle $R_2$ représente un radical alkyle saturé, à chaîne droite ou ramifiée, saturé ou insaturé une ou plusieurs fois et éventuellement substitué par des atomes d'halogènes ou des groupes contenant jusqu'à 5 atomes d'oxygène, ou un radical alcoxyalkyle ou encore un radical (alcoxy-alcoxy)-alkyle contenant jusqu'à 30 atomes d'O et de C,

pour la préparation de médicaments servant à la stimulation de la phagocytose.

10. Composés de formule générale (I) :

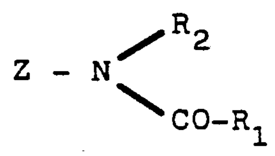 (I)

dans laquelle

Z représente un radical monosaccharide ou un radical maltose relié par l'atome de carbone anomère,

$R_1$ représente un radical alkyle saturé, à chaîne droite ou ramifiée, éventuellement substitué et comportant 9 à 21 atomes de carbone ou un radical alcényle insaturé une ou deux fois et comportant 7 à 21 atomes de carbone, et

$R_2$ représente un radical alkyle ou un radical aralkyle comportant jusqu'à 30 atomes de carbone, à chaîne droite ou ramifiée, saturé ou insaturé une ou plusieurs fois et éventuellement substitué par des atomes d'halogènes ou des groupes contenant jusqu'a 5 atomes d'oxygène, ou un radical alcoxyalkyle ou encore un radical (alcoxy-alcoxy)-alkyle comportant jusqu'à 30 atomes d'O et de C,

en vue d'influencer la défense propre au corps, pour potentialiser l'immunité humorale ou pour stimuler la phagocytose du corps humain ou animal.